# EUROPÄISCHE PATENTANMELDUNG

(11) **EP 1 564 291 A2**
(43) Veröffentlichungstag der Anmeldung: **17.08.2005**
(21) Anmeldenummer: 04024018.6
(22) Anmeldetag: 08.10.2004
(51) Int. Cl.: C12N 15/11, A61K 31/7088, A61P 35/00, C07H 21/00

(54) **Oligonukleotide, diese enthaltende Mittel und deren Verwendung**

(30) Priorität: 08.10.2003 DE 10346721
(71) Anmelder: Kalthoff, Holger Prof.Dr.rer.nat., 24160 Kiel (DE)
(72) Erfinder: Bayer, Ernst, Prof., Dr., 72076 Tübingen (DE); Ketterer, Thomas, Dr., 72070 Tübingen (DE); Kalthoff, Holger, Prof. Dr., 24106 Kiel (DE); Englert, Steffen, Dr., 89610 Oberidschingen (DE)
(74) Vertreter: Biehl, Christian, Dipl.-Phys.

(57) **Zusammenfassung**

Die vorliegende Erfindung betrifft bestimmte Oligonukleotide, pharmazeutische Mittel, die diese Oligonukleotide enthalten, und deren therapeutische Verwendung. Die Oligonukleotide sind insbesondere in der Lage, die Proliferation von Prankreastumoren zu hemmen. Damit besitzen diese Oligonukleotide ein therapeutisches Potential zur Behandlung von Pankreastumoren.

## Beschreibung

Die vorliegende Erfindung betrifft bestimmte Oligonukleotide, pharmazeutische Mittel, die diese Oligonukleotide enthalten, und deren therapeutische Verwendung. Die Oligonukleotide sind beispielsweise in der Lage, die Proliferation von Pankreastumoren zu hemmen. Damit besitzen diese Oligonukleotide ein therapeutisches Potential zur Behandlung humaner Tumoren.

Trotz großer Fortschritte bei der Aufklärung der Kanzerogenese, bleibt die effektive Behandlung von Krebs eines der dringlichsten Probleme der heutigen Medizin. Beispielsweise haben Pankreaskarzinome mit einer 5-Jahres-Überlebensrate von 0,4 % eine extrem schlechte Prognose.

Oligodesoxyribonukleotide (ODN) haben sich in jüngerer Zeit als eine Gruppe vielversprechender Wirkstoffe erwiesen, wobei unterschiedliche Wirkungsmechanismen diskutiert werden. Große Erwartungen sind in die sogenannten Antisense-Oligodesoxyribonukleotide (ASODN) gesetzt worden, welche die Expression eines spezifischen Proteins, z.B. eines Onkogens, unterdrücken sollen.

Trotz dieser großen Erwartungen und dem Nachweis *in vitro,* dass ein bestimmtes Gen weniger exprimiert wird, ist bisher nur ein einziges ASODN als Arzneimittel, Vitravene (ISIS, Carlsbad), gegen die zur Erblindung führende Zytomegalie-Retinitis zugelassen worden. Ausgehend von der ursprünglichen Vorstellung einer sterischen Blockade des entsprechenden ribosomalen Translationsprozesses hat sich mittlerweile ein wesentlich differenzierteres Bild möglicher Wirkungsweisen von Antisense-Oligodesoxiribonukleotiden ergeben.

Darüber hinaus wird auch über sogenannte unspezifische biologische Wirkungen von Oligodesoxyribonukleotide berichtet, die nicht die Sequenz eines ASODN aufweisen.

So sind in der WO 02/26754 neben spezifisch gegen p53 gerichteten ASODN auch Sequenzen aus randomisierten Oligonukleotid-Bibliotheken beschrieben, die eine ähnlich starke proliferationshemmende Wirkung auf Pankreastumorzellen zeigen wie die ASODN. Über eine ähnliche Beobachtung wird auch in Fiedler A. et al., Langenbecks Arch Surg (1998) 383: 269-275 berichtet.

Es wurden nun weitere nicht sequenzspezifische Oligonukleotide gefunden, mit denen die Proliferation humaner Pankreastumoren *in vitro* in Zellkulturen und *in vivo* im orthotopen Xenotransplantationsmodell unterdrückt werden kann. Von Vorteil sind dabei insbesondere solche Oligonukleotide, die in den terminalen 3'- und/oder 5'-Stellungen kovalent gebundene, lipophile Molekülgruppen aufweisen.

Gegenstand der vorliegenden Erfindung sind daher die in den Ansprüchen angegebenen Oligonukleotide.

Erfindungsgemäße Oligonukleotide weisen somit wenigstens einen Teil der Sequenz SEQ ID NO:1 auf. Zusätzlich zur Sequenz SEQ ID NO:1 oder einer Teilsequenz davon können erfindungsgemäße Oligonukleotide weitere Nukleobasen aufweisen, die bei linearen Oligonukleotiden am 3'-Terminus und/oder 5'-Terminus der Sequenz bzw. Teilsequenz angeordnet sind. Diese zusätzlichen Nukleobasen sind im Prinzip frei wählbar, werden aber vorteilhafterweise den hier beschriebenen erfindungsgemäßen Ausführungsformen entsprechend gewählt.

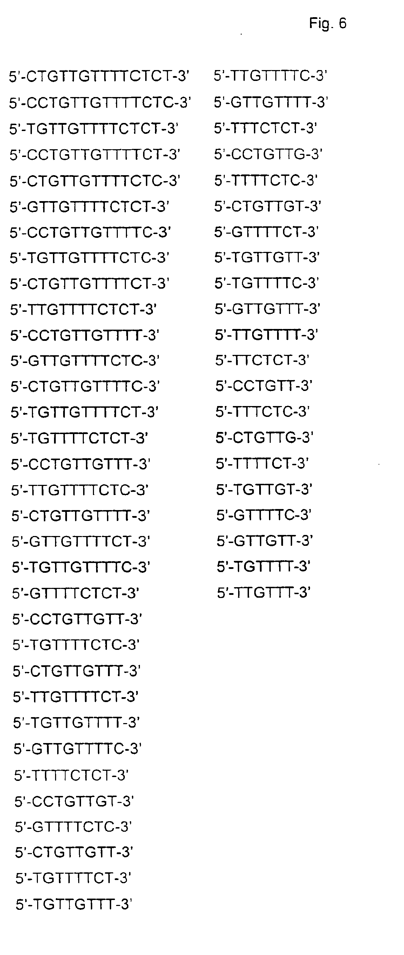

Von der Sequenz SEQ ID NO:1 abgeleitete Teilsequenzen erfindungsgemäßer Oligonukleotide können von unterschiedlicher Länge sein. Im Hinblick auf die erfindungsgemäße Verwendung der Oligonukleotide sollte die Teilsequenz wenigstens 6, insbesondere wenigstens 8, bevorzugt wenigstens 10 und noch bevorzugter wenigstens 12 konsekutiven Nukleobasen aus der Sequenz SEQ ID NO:1 entsprechen. Der Begriff "konsekutiv" wird hier im Sinne von aufeinanderfolgend verwendet. Damit ergeben sich für den Fachmann bestimmte Teilsequenzen der Sequenz SEQ ID NO:1. Einige Teilsequenzen sind in Figur 6 dargestellt. Zum Aufbau erfindungsgemäßer Oligonukleotide kann der Fachmann eine oder mehr als eine, beispielsweise 2, gleiche oder verschiedene dieser Teilsequenzen, gegebenenfalls unter Hinzufügung von zusätzlichen Nukleobasen, in geeigneter Weise anordnen, um so ein erfindungsgemäßes Oligonukleotid zu erhalten.

Der Begriff "Oligonukleotid" meint ein Oligomer aus Ribonukleinsäure (RNA) oder Desoxyribonukleinsäure (DNA) oder Mimetika davon. Hierzu gehören Oligonukleotide, die aus natürlich vorkommenden Nukleobasen, Zuckern und kovalenten Internukleosid-Verknüpfungen (Rückgrat) bestehen, genauso wie Oligonukleotide mit Strukturen, die in der Natur nicht vorkommen, deren Funktion aber den natürlich vorkommenden ähnlich ist. Derartig modifizierte Oligonukleotide werden den nativen Formen erfindungsgemäß vorgezogen, da sie gewünschte Eigenschaften, beispielsweise eine erhöhte zelluläre Aufnahme, eine erhöhte Affinität für Target-Nukleinsäuren und eine erhöhte Stabilität in Gegenwart von Nukleasen aufweisen.

Insbesondere können die Nukleotide Modifizierungen oder Substitutionen an den Nukleobasen (hier auch einfach als "Base" bezeichnet) aufweisen. Zu den nicht modifizierten oder natürlichen Nukleobasen gehören die Purinbasen Adenin (A) und Guanin (G), und die Pyrimidinbasen Thymin (T), Cytosin (C) und Uracil (U). Zu modifizierten Nukleobasen (Mimetika der natürlichen Nukleobasen) gehören synthetische Nukleobasen wie 5-Methylcytosin (5-Me-C), 5-Hydroxymethylcytosin, Xanthin, Hypoxanthin, 2-Aminoadenin, 6-Methyl- und weitere Alkyl-Derivate von Adenin und Guanin, 2-Thiouracil, 2-Thiothymin und 2-Thiocytosin, 5-Halouracil und 5-Halocytosin, 5-Propinyluracil und 5-Propinylcytosin, 6-Azouracil, 6-Azocytosin und 6-Azothymin, 5-Uracil (Pseudouracil), 4-Thiouracil, 8-Halo-, 8-Amino-, 8-Thiol-, 8-Thioalkyl-, 8-Hydroxyl- und weitere 8-substituierte Adenine und Guanine, 5-Halo-, insbesondere 5-Bromo-, 5-Trifluormethyl- und weitere 5-substituierte Uracile und Cytosine, 7-Methylguanin und 7-Methyladenin, 8-Azaguanin und 8-Azaadenin, 7-Deazaguanin und 7-Deazaadenin und 3-Deazaguanin und 3-Deazaadenin. Von diesen Nukleobasen sind bestimmte besonders brauchbar zur Erhöhung der Bindungsaffinität. Hierzu gehören 5-substituierte Pyrimidine, 6-Azapyrimidine und N-2-, N-6- und O-6-substituierte Purine, z.B. 2-Aminopropyladenin, 5-Propinyluracil und 5-Propinylcytosin. Beispielsweise konnte gezeigt werden, dass 5-Methylcytosin die Stabilität eines Nukleinsäure-Duplex um 0,6-1,2°C erhöht.

Erfindungsgemäß geeignet sind T-reiche Oligonukleotide, in denen mehr als 50 %, insbesondere mehr als 60 % und vorzugsweise mehr als 70 % der Nukleobasen Thymidinreste oder Mimetika davon sind. Mimetika von Thymidinresten sind in obigem Sinne modifizierte Nukleobasen, die ähnliche Bindungseigenschaften, also vor allem gleiche Basenpaarungsspezifität, aber durchaus unterschiedliche Affinität für komplemetäre Nukleobasen aufweisen. Analoges gilt für Mimetika anderer Nukleobasen.

Ein im Vergleich zur insbesondere in Säugern und vor allem Menschen vorkommenden natürlichen Basenverteilung erhöhtes Verhältnis von Thymidin oder Mimetika davon zu Cytosin oder Mimetika davon ist erfindungsgemäß zweckmäßig. Ein T/C-Verhältnis von 2:1 oder mehr und vorzugsweise von 3:1 oder mehr ist von Vorteil.

Gemäß einer besonderen Ausführungsform weisen erfindungsgemäße Oligonukleotide wenigstens ein GT- bzw. TG-Motiv auf, d.h. mindestens ein Thymidinrest oder ein Mimetikum davon ist in konsekutiver Abfolge (Sequenz) zu einem Guanin oder einem Mimetikum davon angeordnet.

Ferner ist ein relativ geringer Anteil, vorzugsweise von weniger als 15 % und insbesondere von weniger als 10 % Adenin oder Mimetika davon an der Gesamtsequenz des Oligonukleotids erfindungsgemäß zweckmäßig. Gemäß einer besonderen Ausführungsform enthalten erfindungsgemäße Oligonukleotide eine Anordnung von 6, insbesondere 8, vorzugsweise 10 und noch bevorzugter 12 konsekutiven Basen ohne Adenin. Insbesondere enthalten erfindungsgemäße Oligonukleotide kein Adenin.

In der Regel ist es zweckmäßig, dass die erfindungsgemäßen Oligonukleotide wenigstens 8, vorzugsweise wenigstens 10 und insbesondere wenigstens 12 Nukleobasen aufweisen. Dies ist insbesondere für die Wirksamkeit der Oligonukleotide von Bedeutung. Einem weiteren Aspekt zufolge weisen erfindungsgemäße Oligonukleotide in der Regel weniger als 50, vorzugsweise weniger als 30 und insbesondere weniger als 25 Nukleobasen auf. Dieser Aspekt ist für die Aufnahme der Oligonukleotide in Zellen und damit für die Bioverfügbarkeit von Bedeutung und entspricht darüber hinaus auch Zweckmäßigkeitserwägungen, wonach ein erfindungsgemäßes Oligonukleotid bei gegebener Wirkung möglichst wenig Nukleobasen aufweisen sollte, um die Komplexizität und den Syntheseaufwand so gering wie möglich zu halten. Besonders bevorzugt sind daher Oligonukleotide mit 8 bis 50, insbesondere mit 10 bis 30 und vor allem mit 12 bis 25, beispielsweise mit 13, 14, 15, 16, 17, 18, 19, 20, 21, 22, 23 oder 24 Basen.

Die Anordnung der Nukleobasen in erfindungsgemäßen Oligonukleotiden wird in der Regel dadurch gewährleistet, dass die Nukleobasen in geeigneter Weise miteinander verknüpft sind. In der Regel ergibt sich ein Oligomer mit einer konsekutiven Abfolge von Nukleobasen (Sequenz), die über ein die Hauptkette bildendes Rückgrat miteinander verknüpft sind. Während lineare Oligomere bevorzugt sind, können in bestimmten Fällen auch verzweigte, cyklische oder gar vernetzte Strukturen geeignet sein.

Bei den Oligonukleotiden handelt es sich in der Regel um miteinander verknüpfte Nukleoside, d.h. Base-Zucker-Kombinationen. Der Basenteil der Nukleoside ist normalerweise eine heterozyklische Base, in den meisten Fällen ein Purin oder ein Pyrimidin. Die Nukleoside werden in der Regel durch eine kovalent an den Zuckerteil des Nukleosids gebundene Gruppe miteinander verknüpft. In denjenigen Nukleosiden, die einen Pentofuranosyl-Zucker aufweisen, kann diese Gruppe entweder an die 2'-, 3'- oder 5'-Hydroxylgruppe des Zuckers gebunden sein. In der Regel verknüpfen diese Gruppen kovalent benachbarte Nukleoside miteinander zu einer linearen, oligomeren Verbindung. Die jeweiligen Enden dieser linearen, oligomeren Struktur können wiederum miteinander verbunden werden, um cyclische Strukturen zu bilden. Offene, lineare Strukturen sind allerdings bevorzugt. Innerhalb der Oligonukleotidstruktur bilden die verknüpfenden Gruppen im Allgemeinen das Internukleosid-Rückgrat des Oligonukleotids. Die normale Verknüpfung oder das normale Rückgrat von RNA und DNA sind 3'-5'-Phosphodiester-Verknüpfungen, d.h. die verknüpfenden Gruppen sind Phosphatgruppen.

Erfindungsgemäß bevorzugt sind allerdings Oligonukleotide, die ein modifiziertes Rückgrat bzw. nicht natürliche Internukleosid-Verknüpfungen aufweisen.

Insbesondere gehören zu bevorzugten modifizierten Oligonukleotid-Rückgraten einerseits phosphatfreie Analoga und andererseits Phosphatderivate.

Insbesondere sind Phosphothioate, partielll oder vollständig sulfuriert, z.B. chirale Phosphothioate, Phosphomonothioate und Phosphodithioate zu nennen. Weitere modifizierte Rückgrate sind Phosphotriester, Alkylphosphotriester, Aminoalkylphosphotriester, Methyl- und weitere Alkylphosphonate, z.B. 3'-Alkylenphosphonate und chirale Phosphonate, Phosphinate, Phosphoramidate, z.B. 3'-Aminophosphoramidate und Aminoalkylphosphoramidate, Thionophosphoramidate, Thionoalkylphosphonate, Thionoalkylphosphotriester und Borphosphate mit normalen 3'-5'-Verknüpfungen, 2'-5'-verknüpfte Analoga davon, und solche mit entgegengesetzter Polarität, wobei die benachbarten Paare von Nukleosid-Einheiten 3'-5' zu 5'-3' oder 2'-5' zu 5'-2' verknüpft sind.

Besondere modifizierte Oligonukleotid-Rückgrate ohne Phosphoratom werden im allgemeinen durch kurzkettige Alkyl- oder Cykloalkyl-Internukleosid-Verknüpfungen, die gegebenenfalls auch Heteroatome oder Heterocyklen umfassen können, gebildet. Hierzu gehören diejenigen mit Morpholino-Verknüpfungen (teilweise vom Zuckerteil des Nukleosids gebildet); Siloxan-Rückgrate; Sulfid-, Sulfoxid- und Sulfon-Rückgrate; Formacetyl- und Thioformacetyl-Rückgrate; Methylenformacetyl- und Thioformacetyl-Rückgrate; Alkenenthaltende Rückgrate; Sulfamat-Rückgrate; Methylenimino- und Methylenhydrazino-Rückgrate; Sulfonate- und Sulfonamid-Rückgrate; Amid-Rückgrate; und weitere mit gemischten N-, O-, S- und CH₂-Komponententeilen.

In weiteren besonderen Oligonukleotiden sind sowohl der Zucker als auch die Internukleosid-Verknüpfung, d.h. das Rückgrat natürlicher Nukleotid-Einheiten modifiziert. Eine derartige oligomere Verbindung, d.h. ein Oligonukleotid mit ausgezeichneten Hybridisierungseigenschaften, wird als "Peptide Nucleic Acid" (PNA) bezeichnet. In PNA-Verbindungen ist das Zucker-Rückgrat eines Oligonukleotids durch ein Amid-artiges Rückgrat, insbesondere ein Aminoethylglycin-Rückgrat, ersetzt. Die Nukleobasen sind erhalten und direkt oder indirekt, beispielsweise über einen Methylcarbonyl-Linker, an die Stickstoffatome des Amidteils des Rückgrats gebunden.

Gemäß einer besonders bevorzugten Ausführungsform betrifft die vorliegende Erfindung Oligonukleotide mit Phosphothioat-Rückgraten und ferner Oligonukleoside mit Heteroatom-Rückgraten, die insbesondere auf Struktureinheiten basieren, wie -CH₂-NH-O-CH₂-, -CH₂-N(CH₃)-O-CH₂- [als Methylenmethylimino- oder kurz MMI-Rückgrat bekannt], -CH₂-O-N(CH₃)-CH₂-, -CH₂-N(CH₃)-N(CH₃)-CH₂- oder -O-N(CH₃)-CH₂-CH₂-.

Modifizierte Oligonukleotide können auch eine oder mehrere substituierte Zuckergruppen enthalten. Neben der Variation der 4'-Position, beispielsweise bei den 4'-Thio- und 4'-Aza-Derivaten, kann vor allem die 2'-Position substituiert sein, z.B. mit OH, F, O-, S- oder N-Alkyl, O-S- oder N-Alkenyl, O-, S- oder N-Alkinyl, oder O-Alkyl-O-Alkyl, worin Alkyl, Alkenyl und Alkinyl vorzugsweise substituiertes oder unsubstituiertes C₁-C₁₀-Alkyl oder C₂-C₁₀-Alkenyl bzw. -Alkinyl sind. Insbesondere bevorzugt sind Substituenten, wie O((CH₂)ₙO]ₘCH₃, O(CH₂)ₙOCH₃, O(CH₂)ₙNH₂, O(CH₂)ₙCH₃, O(CH₂)ₙNH₂ und O(CH₂)ₙON[(CH₂)ₙCH₃)]₂, worin n und m ganze Zahlen von 1 bis 10 sind. Zu bevorzugten Modifizierungen gehört eine Alkoxy-Alkoxy-Gruppe, z.B. 2'-Methoxyethoxy (2'-O-CH₂CH₂OCH₃, ebenfalls als 2'-O-(2-Methoxymethyl) oder 2'-MOE bekannt). Zu einer weiteren bevorzugten Modifizierung gehört 2'-Dimethylaminooxyethoxy, d.h. eine O(CH₂)₂ON(CH₃)₂-Gruppe, ebenfalls als 2'-DMAOE bekannt.

Zu weiteren bevorzugten Modifizierungen gehören 2'-Methoxy (2'-O-CH₃), 2'-Aminopropoxy (2'-OCH₂CH₂CH₂NH₂) und 2'-Fluoro (2'-F). Ähnliche Modifizierungen können auch an anderen Positionen des Oligonukleotids, insbesondere an der 3'-Position des Zuckers des 3'terminalen Nukleotids oder in 2'-5'-verknüpften Oligonukleotiden, und an der 5'-Position des 5'-terminalen Nukleotids vorgenommen werden. Ebenfalls können erfindungsgemäße Oligonukleotide Zucker-Mimetika, wie Cyclobutyl-Gruppen anstatt des Pentofuranosyl-Zuckers aufweisen.

Eine weitere bevorzugte Zuckermodifikation ist unter dem Begriff "locked nucleic acid", kurz LNA bekannt. Hierbei sind das C4'-Atom und das C2'-Atom über eine Methylenbrücke miteinander verknüpft, so dass sich ein [2.2.1]-Bicyclo-Nükleosid ergibt.

Weitere Modifikationen erfindungsgemäßer Oligonukleotide beinhalten, dass man an das Oligonukleotid eine oder mehrere Molekülgruppen knüpft, die Aktivität, celluläre Verteilung oder celluläre Aufnahme der Oligonukleotide modifizieren. Zu derartigen Gruppen gehören insbesondere solche, die den lipophilen Charakter der Oligonukleotide erhöhen. Polyglykole, insbesondere Polyalkylenglykole, vorzugsweise Polyethylenglykole, Peptide, Proteine und vor allem lipophile Reste, wie alicyclische Kohlenwasserstoffe, Wachse, Terpene und Fettsäurereste mit vorzugsweise 8 bis 32 Kohlenstoffatomen, die gesättigt oder einfach oder mehrfach ungesättigt sein können, Steroide und vor allem Vitamine sowie Derivate davon sind insbesondere zu nennen. Zu geeigneten Steroidmolekülen gehören insbesondere die Gallensäuren, z.B. Cholinsäure, Desoxycholinsäure und Dehydrocholinsäure, Cortison, Digoxigenin, Testosteron, Cholesterol und kationische Steroide, z.B. Cortison mit einer an die 3-Position des Cortisonrings über eine Doppelbindung gebundenen Trimethylaminomethylhydrazid-Gruppe. Zu geeigneten Vitaminen gehören sowohl wasserlösliche als auch fettlösliche Vitamine. Thiamin, Riboflavin, Nicotinsäure oder Niacin, die Pyridoxal-Gruppe des Vitamins B₆, Pantothensäure, Biotin, Folsäure, die B₁₂-Cobamid-Coenzyme, Inositol, Cholin und Ascorbinsäure sind insbesondere als wasserlösliche Vitamine zu nennen. Erfindungsgemäß bevorzugt sind die fettlöslichen Vitamine, wie die Vitamin-A-Familie, z.B. Retinsäure und Retinol, Vitamin D und dessen Derivate, z.B. Vitamin D und dessen Ergosterol-Vorstufen, die Vitamin-E-Familie, z.B. α-(D,L)Tocopherol und weitere Tocopherole, und Vitamin K und dessen Derivate, z.B. Vitamin K und verwandte Chinon- bzw. Phytolverbindungen.

Ferner gehören zu geeigneten Molekülgruppen auch Konjugate, wie Nanopartikel, die zweckmäßigerweise positiv geladen sind. Durchmesser von 100 bis 500 nm sind brauchbar. Polymere finden in diesem Zusammenhang besondere Verwendung. So sind insbesondere Partikel auf Polystyrol- oder Polyacrylatbasis zu nennen.

Durch die Modifikation mit derartigen Molekülgruppen vor allem in 3'- und 5'-Stellung kann die Bioverfügbarkeit wegen der um Größenordnungen verlängerten Halbwertszeit gegenüber dem Angriff von Nukleasen erhöht werden. Konjugiert man die erfindungsgemäßen Oligonukleotide an positiv geladene Nanopartikel, wird ebenfalls die Stabilität gegenüber dem Angriff von Nukleasen sowohl bei nicht modifizierten, Phosphothioat-Verknüpfungen aufweisenden, als auch terminal modifizierten Oligonukleotiden erhöht und eine vorzügliche Aufnahme in Tumorzellen beobachtet.

Erfindungsgemäß besonders bevorzugt sind Oligonukleotide, die terminal modifiziert sind. Zu terminal modifizierten Oligonukleotiden gehören insbesondere solche, die keine freie 3'-OH-Gruppe und/oder 5'-Phosphat-Gruppe aufweisen. Dementsprechend sind erfindungsgemäße Oligonukleotide am 3'- und/oder 5'-Terminus modifiziert. Zu diesem Zweck eignen sich insbesondere die oben beschriebenen Molekülgruppen, wobei Polyalkylenglykole, lipophile Reste und Peptide bevorzugt sind. Von den Polyalkylenglykolen sind insbesondere Polyethylenglykole, beispielsweise Hexaethylenglykol oder solche mit einer mittleren Molmasse von 150 bis 5000 Dalton zu nennen. Von den lipophilen Resten sind insbesondere Fettsäurereste, Steroidreste und Vitaminreste sowie deren Derivate zu nennen. Besonders bevorzugt sind Reste fettlöslicher Vitamine wie Tocopherole.

Gemäß einer besonderen Ausführungsform weisen erfindungsgemäße Oligonukleotide terminal gebundenes α-Tocopheryl, vorzugsweise α-D-Tocopheryl und/oder Polyethylenglykol auf. Insbesondere können erfindungsgemäße Oligonukleotide im Rahmen dieser Ausführungsform an einem Terminus einen Polyethylenglykolrest und am anderen Terminus einen Tocopherylrest aufweisen, wobei es bevorzugt ist, dass der Polyethylenglykolrest am 3'-Terminus und der Tocopherylrest am 5'-Terminus gebunden ist. Besonders bevorzugt sind im Rahmen dieser Ausführungsform erfindungsgemäße Oligonukleotide, die an beiden Termini einen Tocopherylrest aufweisen.

Die terminalen Reste können im Prinzip beliebig an das Oligonukleotid gebunden sein. Aufgrund struktureller Vorgaben durch das Oligonukleotid und ihrer relativ einfachen synthetischen Zugänglichkeit bieten sich allerdings Amid-, Ester- oder Etherbindungen insbesondere für die 3'- und 5'-Termini an. Die Bindung an die Termini kann die direkt oder über geeignete Linkerverbindungen, die gegebenenfalls den unten erläuterten Ankerverbindungen oder Teilen davon entsprechen können, erfolgen.

Zudem können weitere Modifizierungen erfindungsgemäßer Oligonukleotide einzelne Nukleoside betreffen, wobei mehrere Nukleoside unterschiedlich oder einheitlich modifiziert sein können. Auch Gemische aus unterschiedlich modifizierten Oligonukleotiden sind insbesondere im Hinblick auf die erfindungsgemäße Anwendung erfindungsgemäß brauchbar.

Brauchbar sind ebenfalls pharmazeutisch verträgliche Salze, Ester, Salze der Ester oder beliebige weitere Verbindungen, die nach Verabreichung an ein Tier, insbesondere Menschen, in der Lage sind, direkt oder indirekt den biologisch aktiven Metaboliten oder einen Teil davon, zu liefern.

So sind beispielsweise auch sogenannte "Prodrugs" brauchbar. Hierbei handelt es sich um eine inaktive Form des Wirkstoffs, die in-vivo in eine aktive Form umgewandelt wird. Prodrug-Versionen von Oligonukleotiden können beispielsweise als S-(Acetyl-2-thioethyl)phosphat-Derivate bereitgestellt werden.

Zu den pharmazeutisch verträglichen Basenadditionssalzen gehören Salze mit Metallen oder Aminen, wie Alkali- und Erdalkalimetallen oder organischen Aminen, insbesondere Kationen wie Natrium, Kalium, Ammonium, Magnesium und Calcium bzw. Polyaminen wie Spermin und Spermidin.

Zu den pharmazeutisch akzeptablen Säureadditionssalzen gehören Salze mit anorganischen Basen, z.B. Chlorwasserstoffsäure, Bromwasserstoffsäure, Schwefelsäure, Phosphorsäure, Salpetersäure und dergleichen, sowie mit organischen Säuren, z.B. Essigsäure, Oxalsäure, Weinsäure, Bernsteinsäure, Maleinsäure, Fumarsäure, Gluconsäure, Zitronensäure, Äpfelsäure, Ascorbinsäure, Benzoesäure, Tanninsäure, Palmitinsäure, Algininsäure, Polyglutaminsäure, Naphthalinsulfonsäure, Methansulfonsäure, p- Toluolsulfonsäure, Naphthalindisulfonsäure, Polygalactoronsäure und dergleichen.

Die erfindungsgemäßen Verbindungen können in an sich bekannter Art und Weise hergestellt werden. Insbesondere finden bekannte Festphasensynthese-Systeme, wie die Phosphoramidit-Methode, Anwendung. Geeignete Vorrichtungen sind kommerziell erhältlich, beispielsweise von Applied Biosystems (Foster City, Kalifornien). Demnach werden die Verbindungen in-vitro synthetisiert.

Auch die Synthese von sulfurierten Oligonukleotiden (Phosphothioaten) kann in Anlehnung an die Phosphoramidit-Chemie erfolgen. Im Unterschied zur Herstellung von Phosphodiestern wird anstelle der Oxydation des dreiwertigen Phosphors ein Sulfurierungsschritt durchgeführt. Hierfür eignen sich eine Vielzahl bekannter Sulfurierungsmittel, wobei 3H-1,2-Benzodithiol-3-on-1,1-dioxid, das sogenannte Beaucage-Reagens, üblicherweise und ferner Tetraethylthiuramdisulfid (TETD) häufig verwendet werden. Auch Phenylacetyldisulfid, das sogenannte Van-Boom-Reagens, hat sich als günstig erwiesen. Neben der Phosphoramidit-Chemie kann auch die allgemeine Phosphonat-Methode in entsprechend modifizierter Form zur Herstellung von Phosphothioaten verwendet werden.

Auch die Modifizierung der Oligonukleotide mit Molekülgruppen kann in an sich bekannter Weise erfolgen. Je nach Anknüpfungspunkt stehen verschiedene Methoden bereit, um insbesondere an Base, Zucker und/oder Phosphat-Rückgrat Modifizierungen vomehmen zu können.

Für die terminale Modifizierung am 3'-Terminus bieten sich grundsätzlich zwei Möglichkeiten an. Die Modifizierung kann nach Beendigung der eigentlichen Synthese, also nach der Abspaltung des Oligonukleotids vom Trägermaterial und der Entfernung der Schutzgruppen, an das Oligonukleotid erfolgen. Alternativ kann man Trägermaterialien verwenden, die mit trifunktionellen Ankerverbindungen ausgerüstet sind. Diese Ankerverbindungen weisen neben einer spaltbaren Bindungsstelle zum Trägermaterial (erstes Reaktionszentrum) und einer gegebenenfalls geschützten Hydroxygruppe, an der das Oligonukleotid schrittweise aufgebaut wird (zweites Reaktionszentrum), eine weitere Funktionalisierung, üblicherweise eine Hydroxy- oder Aminogruppe, auf (drittes Reaktionszentrum), an welche die einzuführende Gruppe gebunden werden kann. Letzteres kann prinzipiell vor oder nach der Oligonukleotid-Synthese erfolgen, wobei erforderlichenfalls eine geeignete säurestabile Schutzgruppe, z.B. Fmoc am dritten Reaktionszentrum verwendet wird. Diese kann nach der Oligonukleotid-Synthese in an sich bekannter Weise abgespalten werden. So kann das Oligonukleotid schließlich vom Trägermaterial abgespalten werden, wobei der Oligonukleotid und die einzuführende Molekülgruppe verknüpfende Teil der Ankerverbindung erhalten bleibt.

Die Struktur geeigneter Ankerverbindungen ist weitgehend variabel. Zu beachten ist, dass die Ankerverbindung und deren Bindungen an die einzuführende Molekülgruppe mit der für die Synthese des Oligonukleotids verwendeten Chemie kompatibel sein sollte. Da üblicherweise die Amidit-Chemie zur Anwendung kommt, sollten die Bindungen weder säure- noch basenlabil sein, weshalb Ester- und Amidbindungen zwischen der Ankerverbindung und der einzuführenden Molekülgruppe nur bedingt geeignet sind, wohingegen Etherbindungen bevorzugt werden.

Geeignete Ankerverbindungen sind an sich bekannt. Beispielsweise sind Glycerinderivate als C₃-Anker zu nennen. Hierbei dienen die Hydroxylgruppen in 1- und 3-Position zur Bindung der einzuführenden Molekülgruppe bzw. des Oligonukleotids und die Hydroxylgruppe in 2-Position bildet den Anknüpfungspunkt zum Trägermaterial über eine spaltbare Bindung, die erforderlichenfalls mit Hilfe eines geeigneten Spacers zwischen Hydroxylgruppe und Trägermaterial etabliert werden kann. Eine weitere Variante sind 2-substituierte 1,3-Propandiol-Derivate. Hierbei dienen die Hydroxyl-Gruppen in 1- und 3-Position zur Bindung des Oligonukleotids bzw. des Trägermaterials. Der Substituent in 2-Position dient zur Bindung der einzuführenden Molekülgruppe. Beispielsweise kann man mit Amino-C₁₋₆-Alkylresten Amid- oder Urethanbindungen ausbilden. Ein bevorzugtes Beispiel für eine derartige Ankerverbindung ist 2-(4-Aminobutyl)-1,3-propandiol.

In bestimmten Fällen weisen die einzuführenden Molekülgruppen selbst Hydroxylgruppen auf, an denen sich das Oligonukleotid synthetisieren lässt. Dies ist beispielsweise bei Alkylenglykolen der Fall. Diese können daher zunächst in geeigneter Weise an das Trägermaterial gebunden werden. Dabei sind die als Anknüpfungspunkt für das Oligonukleotid dienenden Hydroxylgruppen erforderlichenfalls in geeigneter Weise, beispielsweise mit Fmoc-Gruppen, geschützt. Die Abspaltung dieser Schutzgruppen stellt anschließend die zur Oligonukleotid-Synthese erforderlichen Hydroxylgruppen bereit.

Geeignete Trägermaterialien sind an sich bekannt und entsprechen im Wesentlichen den üblichen, zur Festphasensynthese von Oligonukleotiden verwendeten Trägern.

Am 5'-Terminus können Molekülgruppen zweckmäßigerweise über entsprechend modifizierte Monomere, also insbesondere Phosphoramidite, eingeführt werden. Diese in an sich bekannter Weise zugänglichen Amidite werden als letztes Monomer an das synthetisierte Oligonukleotid angekoppelt.

Ein erfindungsgemäßes Beispiel für eine hervorragende Hemmung der Proliferation von humanen Pankreastumorzellen ist das Oligonukleotid mit folgender Sequenz:

5`-CCT GTT GTT TTC TCT-3`        (SEQ ID NO:1)

Insbesondere von Vorteil sind die entsprechenden Phosphothioate. Auch die Modifizierungen in 3'-Stellung mit Polyethylenglykol (z.B. Hexaethylenglykol und insbesondere PEG mit einer mittleren Molmasse von 1500) oder mit α-Tocopherol, und in 5'-Stellung mit α-Tocopherol bringen zusätzliche Vorteile, so dass folgende Oligonukleotide besonders bevorzugt sind:

Tocopheryl-5'- CCT GTT GTT GTT TTC TCT-3'-PEG;

Tocopheryl-5'- CCT GTT GTT GTT TTC TCT-3'-Tocopheryl.

Die Sequenz SEQ ID NO:1 ist ein Beispiel für eine aus einer randomisierten Oilgonukleotidbiblïothek ausgewählte Sequenz. Weitere Sequenzen können aus randomisierten Oilgonukleotidbibliotheken im Rahmen der Erfindung ausgewählt und gegebenenfalls modifiziert werden, indem man insbesondere eines oder mehrere der folgenden Selektionskriterien anwendet:
- Hemmung der Proliferation des Wachstums von Pankreastumorzellen durch Bestimmung des ³H-Thymidineinbaus;
- Stabilität der Konstrukte gegen den Angriff von endo- und/oder exo-Nukleasen durch Messung der Halbwertszeit der Oligonukleotide mittels Kapillarelektrophorese;
- Bestimmung der Aufnahme der Oligonukleotide in Zellen durch konfokale Laser-Scanning-Mikroskopie;
- Apoptose-Induktion.

Ein weiterer Gegenstand der vorliegenden Erfindung ist die Verwendung einer erfindungsgemäßen Verbindung zur Proliferationshemmung von Tumorzellen.

Diese Verwendung beinhaltet auch ein Verfahren zur Proliferationshemmung von Tumorzellen, wobei man wenigstens ein erfindungsgemäßes Oligonukleotid auf Tumorzellen einwirken lässt. Grundsätzlich kann es sich bei diesem Verfahren um ein in-vitro-, ex-vivooder in-vivo-Verfahren handeln. Entsprechende Systeme, z.B. in Form von Zellen oder Geweben, können in an sich bekannter Weise in-vitro- oder ex-vivo als Kultur bereitgestellt werden. Eine in-vivo-Anwendung beinhaltet in der Regel die Verabreichung des Oligonukleotids an das betreffende Individuum.

Die Bereitstellung derartiger Tumorzellsysteme kann in herkömmlicher Weise durch den Fachmann erfolgen. Insbesondere sind Techniken bekannt, um Organismen und Zellen zur entsprechenden Entartung, beispielsweise über rekombinante Techniken, zu veranlassen. Es ist auch geläufiges Fachwissen, Organismen, Zellen oder Gewebe in geeigneter Weise zu kultivieren und insbesondere die Proliferation anhand geeigneter Assays qualitativ und gewünschtenfalls auch quantitativ zu beurteilen. Insbesondere ist es eine Sache der Optimierung des Verfahrens, geeignete Bedingungen zu wählen, unter denen das Einwirken erfindungsgemäßer Oligonukleotide eine Modulation der Proliferation bewirkt.

Auch die Verabreichung erfindungsgemäßer Oligonukleotide an ein Individuum lässt sich mit geläufigem Fachwissen bewerkstelligen. In der Regel wird dem Individuum eine bestimmte Menge wenigstens eines erfindungsgemäßen Oligonukleotids, in der Regel der pharmazeutischen oder tierarzneilichen Praxis entsprechend formuliert, verabreicht.

Demgemäß betrifft die vorliegende Erfindung auch Mittel und insbesondere pharmazeutische Mittel, die wenigstens ein erfindungsgemäßes Oligonukleotid sowie gewünschtenfalls geeignete Hilfsstoffe, die insbesondere eine pharmazeutisch verträgliche Formulierungsgrundlage bilden, umfassen. Die Herstellung derartiger Mittel ist geläufiges Fachwissen.

Die erfindungsgemäßen Oligonukleotide können im Rahmen dieser Verwendungen und Verfahren beispielsweise wissenschaftlichen Zwecken dienen. Vor allem können die erfindungsgemäßen Oligonukleotide für therapeutische Zwecke verwendet werden.

Therapeutische Zwecke von besonderer Bedeutung betreffen die Behandlung von Tumoren. Behandlung meint in diesem Zusammenhang präventiv bzw. prophylaktisch vermeiden oder zumindest zeitlich verzögern, bzw. akut oder chronisch lindern oder beheben, d.h. insbesondere die Proliferation des Tumors verringern und gegebenenfalls unterdrücken und damit die Tumorausdehnung verringern sowie das Risiko bzw. die Inzidenz für Metastasen, die sich vom Primärtumor ableiten, senken.

Gegenstand der vorliegenden Erfindung ist daher auch die Verwendung wenigstens eines erfindungsgemäßen Oligonukleotids zur Behandlung von Tumoren. Diese Verwendung beinhaltet auch ein Verfahren, bei dem eine wirksame Menge wenigstens eines erfindungsgemäßen Oligonukleotids einem zu behandelnden Individuum, insbesondere einem Menschen und auch einem Nutz- oder Haustier, verabreicht wird. Die Verabreichung erfolgt in der Regel einmalig oder mehrmalig täglich, gegebenenfalls zusammen oder im Wechsel mit anderen Wirkstoffen oder wirkstoffhaltigen Präparaten. In diesem Sinne kann die Therapie die Anwendung weiterer Behandlungsmaßnahmen miteinschließen, z.B. Chemotherapie durch Verabreichung von Cytostatika oder Strahlentherapie.

Besondere Vorteile ergeben sich bei der Behandlung von Pankreastumoren insbesondere beim Menschen.

Die vorliegende Erfindung wird nun anhand der nachfolgenden Beispiele weiter erläutert.

In den Zeichnungen zeigt
- Figur 1: ein Balkendiagramm, in dem die anhand des ³H-Thymidin-Einbaus gemessene Proliferationshemmung bei Einwirkung der Oligonukleotide MON1 (erfindungsgemäß) sowie MON2 und MON3 (Vergleich) auf PancTu1-Zellen nach 24 h (Figur 1a) und 48 h (Figur 1 b) bezogen auf unbehandelte PancTu1-Zellen dargestellt ist;
- Figur 2: ein Balkendiagramm, in dem die anhand des ³H-Thymidineinbaus gemessene Proliferationshemmung bei Einwirkung des Oligonukleotids MON1 (erfindungsgemäß) auf PancTu1-, Capan1-, colo357- und A818-4-Zellen nach 24 h (Figur 1 a) und 48 h (Figur 1b) bezogen auf unbehandelte PancTu1 -Zellen dargestellt ist;
- Figur 3: ein Balkendiagramm, in dem das mittlere Gewicht des Primärtumors, der im Anschluss an die angegebene Behandlung mit MON10 Mäusen (n = 42) mit orthotopen PancTu1-Tumoren entnommen wurde, dargestellt ist;
- Figur 4: ein Balkendiagramm, in dem das mittlere Gewicht von Gesamttumorgewebe, das im Anschluss an die angegebene Behandlung mit MON10 Mäusen (n = 42) mit orthotopen PancTu1-Tumoren entnommen wurde, dargestellt ist;
- Figur 5: ein Balkendiagramm, in welches das mittlere Gewicht der Milz, die im Anschluss an die angegebene Behandlung mit MON10 Mäusen mit orthotopen PancTu1-Tumoren entnommen wurde, dargestellt ist.
- Figur 6: eine Auflistung von Teilsequenzen der Sequenz SEQ ID NO:1 mit 6 bis 14 Nukleobasen.

### Methodischer Teil

| Geräte | |
|---|---|
| Oligonukleotidsynthesizer: | 394 DNA/RNA Synthesizer (Applied Biosystems) 392 DNA/RNA Synthesizer (Applied Biosystems) |
| Massenspektrometer: | ES-MS:API III TAGA 6000E Triple Quadrupol |
| | Massenspektrometer (Sciex, Perkin Elmer Corp.) FD-MS: MAT 711 A, TSQ 70 (Finnigan) |
| HPLC-Anlage | Analytisch: S8110, S1100 (Sykam), UVIS 205 (Linear) Datensystem: Pyramid Chromatography Manager Axxiom) Präparativ: S8110, S1020, S 2000 (Sykam), UVIS 200 (Linear) |
| HPLC-Säulen | Nucleosil C18 5µ 250x4,6 mm (Grom) Gromsil ODS-2 FE 5µ 250x4,6 mm (Grom) Gromsil ODS-2 FE 5µ 250x25 mm (Grom) |
| Kapillarelektrophorese | Kapillarelektrophorese-System Beckman P/ace System MDQ |
| UV-Spektrometer | Lamda 5 (Perkin Elmer) |
| NMR-Geräte | WT 80. Avante DRX 250, AC 250, WM 400 (Bruker) |
| Zentrifuge | EBA 12 R (Hettich) |
| Reinstwasseranlage | MilliQ185 Plus (Millipore) |
| Rundschüttelmaschine | Eigenbau des Chemischen Instituts (Universität Tübingen) |
| Lyophilisatoren | Gefriertrocknungsanlage Delta II (Christ) Gefriertrocknungsanlage Alpha 1-2 (Christ) |
| Vortexer | Vibrofix VF1 Electronic (Janka und Kunkel) |
| Ultraschallbad | Elma T 760 DH |

| Chemikalien und Materialien | |
|---|---|
| Laborchemikalien zur Synthese | Aldrich, Fluka, Merck, Sigma |
| Chemikalien zur DNA-Synthese | Proligo Biochemie GmbH, Applied Biosystems |
| Nukleotidsuccinate | Sigma |
| TETD (Sulfurierungsreagenz) | Applied Biosystems |
| Beaucage (Sulfurierungsreagenz) | Eurogentec |
| Trägermaterialien für | PS-PEG-Amin (TentaGel S): Rapp |
| Festphasensynthesen | Polymere CPG: Proligo Biochemie GmbH Primer Support (PS hochvernetzt): Amersham Pharmacia Biotech |
| Acetonitril (HPLC) | Merck LiChrosolv, gradient grade |
| DMF | J.T. Baker, Merck |
| Dioxan | Merck, reinst |
| Methanol | Merck, reinst |
| Ethanol | Merck, p.a. |
| 25 % Ammoniaklösung | Fluka, p.a. |
| Thiol- und Amino-Modifier | Eurogentec |
| 5'-Cy3-Markierung | Cy3-Amidit-Eurogentec |
| Cy3 Aktivester | FluoroLink™: Amersham Pharmacia Biotech |
| Tocopherol | Fluka |
| 2-Cyanothyl-N,N-diisopropylamino-phosphinchlorid | Sigma |
| Dünnschichtchromatographie (DC)-Platten | Kieselgel Fertigplatten 60 F₂₅₄: Merck |

### Synthesen

### Allgemein

Die modifizierten ODN werden an einem Applied Biosystem Modell 394 DNA/RNA Synthesizer mit TentaGel®N (Copolymer auf Basis einer quervernetzten Polystyrol-Matrix mit etwa 50-70 Gew.-% über Benzylether-Verknüpfungen aufgepfropftem Polyethylenglycol; erhältlich von Rapp Polymere Tübingen) als Träger hergestellt. Modifizierte Protokolle für die Synthese an Tentagel werden entsprechend der Literatur (E. Bayer, M. Maier, K. Bleicher, H.-J. Gaus, Z. Naturforsch. 50b, 671-676 (1995) verwendet. Als Reagenzien werden dabei folgende Lösungen eingesetzt: 3 % Trichloressigsäure (TCA) in 4-(Dicyanmethylen)-6-(4-dimethylaminostyryl)-2-methyl-4H-pyran (DCM) zur Abspaltung der 4,4'-Dimethoxytriphenylmethyl (DMT)-Schutzgruppe, Amidite in einer Konzentration von 0,1 M in Acetonitril sowie 0,25 M DCI in Acetonitril zur Kopplung, ein Acetanhydrid/Lutidin/1-Methylimidazol-Gemisch in Tetrahydrofuran (THF) als Capping-Komponente und zur Sulfurierung TETD oder Beaucage-Reagenz in Acetonitril gelöst (0,05-0,2 M).

PEG, Fettsäurereste, Cholesteryl- und Tocopherylrste am 5'-Ende wurden als Phosphoramidite in CH₂Cl₂ bzw. Acetonitril (0,1 M) eingeführt. Sollen ODN mit einem Phosphodiester-Rückgrat hergestellt werden, wird zur Oxidation 0,1 M lod in Pyridin/THF/H₂O verwendet und anschließend ein Waschschritt mit 0,1 M Ascorbinsäure in Dioxan/H₂O (9:1) eingefügt, um überschüssiges lod zu entfernen. Sind Amidite, die zur Lipidmodifizierung eingesetzt werden, in Acetonitril nicht ausreichend löslich, (z.B. das Tocopherylamidit), kann DCM als Lösungsmittel verwendet werden.

Allerdings müssen dann die Leitungen vor und nach der Kopplung mit DCM gespült werden, um ein Ausfallen des Amidites zu verhindern. Eine zusätzliche Verlängerung der Kopplungszeiten ist beim Tocopherylamidit nicht erforderlich. Die Abspaltung der ODN vom Trägermaterial sowie die Hydrolyse der Schutzgruppen erfolgt in konzentriertem Ammoniak (25 %) bei 55 °C (7-8 h). Die Reinigung der DMT-geschützten ODN's wird mit HPLC durchgeführt: 15 min. linearer Gradient 20-80 % Acetonitril in 0.1 M Triethylammoniumacetat, Fluß 1 ml/min, Säule Nucleosil 100 C18, 250x4 mm, bzw. Fluß 15 ml/min, Säule GROM-SIL 100 ODS2 FE, 250x20 mm (Grom, Herrenberg).

### 3'-PEG-ODN

### 1-(4,4'-Dimethoxytrityl)-polyethylenglykol (DMT -PEG₁₅₀₀)

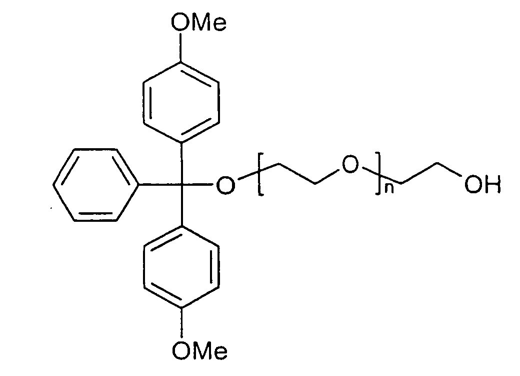

21 g PEG₁₅₀₀ (14 mmol) werden zur Trocknung zweimal in je 30 ml absolutem Pyridin gelöst, das anschließend azeotrop abdestilliert wird. Das getrocknete Edukt wird in 35 ml absolutem Pyridin gelöst. Zu dieser Lösung werden über einen Zeitraum von 30 min 4,7 g in 35 ml absolutem Pyridin gelöstes 4,4'-Dimethoxytritylchlorid (13,9 mmol) zugetropft. Es wird weitere 2 h bei RT gerührt und dabei der Reaktionsverlauf mittels Dünnschichtchromatographie (DC) kontrolliert. Neben des Nachweises der DMT-Gruppe mittels UV-Lampe können die DC-Platten in zwei Schritten entwickelt werden: 1. in HCl-gesättigter Atmosphäre zum Nachweis von DMT; 2. in der lodkammer zum Nachweis von PEG; PEG kann außerdem mit dem Dragendorff-Bürger-Sprühreagenz nachgewiesen werden. Nach Beendigung der Reaktion wird das Lösungsmittel entfernt und das Produkt in 50 ml DCM aufgenommen. Die organische Phase wird zweimal mit je 25 ml 5 % NaHCO₃-Lösung sowie zweimal mit je 25 ml H₂O gewaschen. Die Phasentrennung zwischen wässriger und organischer Phase ist dabei langwierig, da PEG sowohl hydrophoben als auch hydrophilen Charakter besitzt. Nach dem Trocknen über Na₂SO₄ wird das Lösungsmittel entfernt und das Rohprodukt säulenchromatographisch über Kieselgel mit DCM/MeOH/TEA = 18:2:0,5 gereinigt. Die produkthaltigen Fraktionen werden mittels DC identifiziert, vereinigt und zur Trockne eingeengt. Man erhält ein gelbliches Öl, das nach dem Trocknen im Hochvakuum zu einem wachsartigen Feststoff wird.
Ausbeute: 18,3 g (72,5 % der Theorie)
DC (DCM/MeOH/TEA = 18:2:0,5): R_{f}-Wert 0,55 (durch die Molmassenverteilung von PEG verbreitertes Signal)

### 1-(4,4'-Dimethoxytrityl)-hexaethylenglykol (DMT-HEG)

10 g Hexaethylenglykol (HEG; 35 mmol) werden durch Coevaporation mit zweimal je 30 ml absolutem Pyridin getrocknet und anschließend in 20 ml absolutem Pyridin gelöst. Analog zur Vorschrift für DMT-PEG₁₅₀₀ wird das HEG mit 10 g in 50 ml absolutem Piridin gelöstem DMT-Cl (29,5 mmol) umgesetzt. Die säulenchromatigraphische Aufreinigung erfolgt mit Ethylacetat /TEA = 95:5. Als getrocknetes Produkt erhält man ein zähes gelbes Öl. Ausbeute: 12,5 g (60,5 % der Theorie)
DC (DCM/MeOH = 95,5): R_{f}-Wert = 0,59
MS (FD): m/z 583,9 (M⁺)
¹H-NMR (CDCl₃): δ 3,21 (t, DMT-OCH₂), 3,47-3,68 (m, -CH₂-), 3,76 (s, -CH₃), 6,77-7,46 (m, Aromaten)

### 1-(4,4'-Dimethoxytrityl)-polyethylenglykolsuccinat (DTM-PEG-Suc)

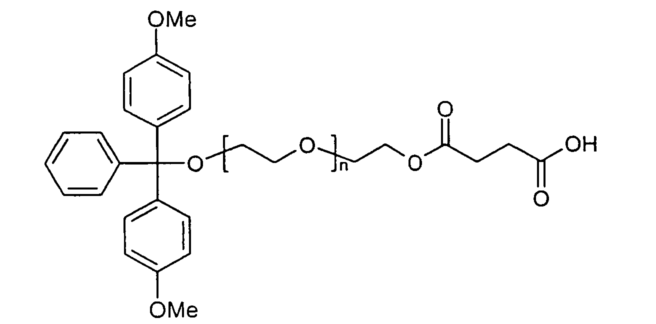

Nachstehende Arbeitsvorschrift ist sowohl für DMT-PEG₁₅₀₀ als auch für DMT-HEG anwendbar.

5 g DMT-PEG₁₅₀₀ (2,8 mmol) werden in 25 ml DCM/Pyridin = 5:1 gelöst und mit 420 mg in 7 ml Pyridin gelöstem Bernsteinsäureanhydrid (4,2 mmol, d.h. 1,5 eq) sowie mit 170 mg in 3 ml Pyridin gelöstem Dimethylaminopyridin (DMAP) (1,4 mmol, d.h. 0,6 eq) versetzt. Nach 12 h Rühren bei RT werden die Lösungsmittel im Vakuum abgezogen und der Rückstand in DCM aufgenommen. Die organische Phase wird gründlich dreimal mit NaHCO₃-Lösung (10 % in H₂O) und zweimal mit gesättigter wässriger NaCl-Lösung gewaschen, um die überschüssige Bernsteinsäure abzutrennen. Nach dem Trocknen über Na₂SO₄ wird das Lösungsmittel entfernt. Die Succinate können nach gründlicher Trocknung am Hochvakuum ohne weitere Aufarbeitung zur Kopplung auf aminomodifizierte Trägermaterialien eingesetzt werden.

### DC: DMT-PEG₁₅₀₀-Suc (DCM/MeOH/TEA = 18:2:0,5): R_{f}-Wert = 0,41 DMT-HEG-Suc (DCM/MeOH = 9:2): R_{f}-Wert = 0,70

Beladung von PS-PEG Trägermaterial mit DMT-PEG₁₅₀₀Suc

Bei der Beladung von PS-PEG Trägermaterial mit DMT-PEG₁₅₀₀-Suc wird mit Überschüssen gearbeitet. Die Beladung kann über die eingesetzte Menge Succinat gesteuert werden. Nachstehend ein Beispielprotokoll für die Derivatisierung von Tentagel® mit DMT-PEG₁₅₀₀.

Man lässt 3 g Tentagel (0,25 mmol/g) in 20 ml DCM quellen. 1,5 mmol DMT-PEG₁₅₀₀-Suc (2,85 g) werden in 15 ml DCM gelöst und zum Harz gegeben. 468 mg O-(Benzotriazol-1-yl)-N,N,N',N'-tetramethyluroniumtetrafluorborat (TBTU) (1,5 mmol) werden in ca. 7 ml DMF gelöst und ebenso wie 330 µl N-Methylmorpholin (NMM) (3 mmol) zum Reakktionsgemisch gegeben. Die Mischung wird lichtgeschützt 12 h geschüttelt. Anschließend wird das Herz abfiltriert und gründlich mit DMF, DCM und Methanol gewaschen und im Vakuum getrocknet. Nicht umgesetzte Aminofunktionen auf dem Harz können sowohl manuell als auch am DNA-Synthesizer acetyliert werden. Die Vollständigkeit der Kopplung kann mit einem Kaisertest überprüft werden. Die Beladung kann spektrometrisch über DMT-Abspaltung bestimmt werden. Es ergibt sich eine Beladung von 75 µmol/g.

### 3'-Tocopheryl-ODN

### 1 -Tosyl-2,3-isopropylidenglycerol

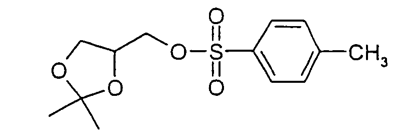

200 mmol Isopropylidenglycerol (26,4 g) werden in 200 ml Acetonitril gelöst und mit 22,2 g Triethylamin (220 mmol) versetzt. 220 mmol p-Toluolsulfonsäurechlorid (41,9 g) werden in 250 ml Acetonitril gelöst und unter Rühren über 2 h zum Reaktionsgemisch zugetropft. Man lässt weitere 20 h bei RT rühren, wobei ein weißer Niederschlag ausfällt, welcher nach Beendigung der Reaktion abfiltriert wird. Das Lösungsmittel wird entfernt und das Rohprodukt über Kieselgel säurenchromatographisch mit n-Hexan/Ethylacetat = 2:1 aufgereinigt. Die produkthaltigen Fraktionen werden mittels DC identifiziert, vereinigt und zur Trockne eingeengt. Das Produkt wird am Hochvakuum getrocknet. Es resultiert ein gelbliches Öl.
Ausbeute: 31,3 g (54,7 % der Theorie)
DC (n-Hexan/Ethylacetat = 2:1): R_{f}-Wert = 0,2
MS (FD): m/z 272,0 (M⁺-CH) (286,3 berechnet)
¹H-NMR (CDCl₃): δ 1,31 (s); 1,34 (s); 2,45 (s); 3,74-3,79 (m); 3,90-4,08 (m); 4,23-4,32 (m); 7,36 (d); 7,77 (d)
¹³C-NMR: δ 21,7; 25,2; 26,7 (Methyl-C); 66,2; 69,5; 72,9 (Glycerin-C); 110,1 (Methylen-C); 128,0; 129,9; 132,7; 145,1 (Aromaten-C)

### 2,3-Isopropyliden-1-D,L-α-tocopherolglycerol (Toc1)

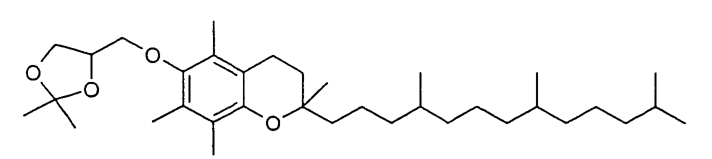

Zu 56 mmol D,L-α-Tocopherol (24,06 g) in 280 ml DMSO werden 5,6 g pulverisiertes Kaliumhydroxid (100 mmol) zugegeben. Nach 2 h Rühren bei RT unter Lichtausschluss werden 56 mmol in 20 ml DMSO gelöstes 1-Tosyl-2,3-isopropylidenglycerol (16 g) zugetropft und weitere 12 h bei 60 °C gerührt. Danach wird das Reaktionsgemisch auf 1 I Eiswasser hydrolysiert und die wässrige Phase mit 1,5 I Toluol extrahiert. Nachdem über Natriumsulfat getrocknet wurde, wird das Lösungsmittel entfernt. Das Rohprodukt wird über Kieselgel säulenchromatographisch mit n-Hexan/Ethylacetat = 1:1 aufgereinigt. Die produkthaltigen Fraktionen werden mittels DC identifiziert, vereinigt und zur Trockne eingeengt. Das Produkt wird im Hochvakuum getrocknet und unter Lichtausschluss aufbewahrt. Es resultiert ein gelbliches Öl.
Ausbeute: 24,2 g (79,2 % der Theorie)
DC (n-Hexan/Etzylacetat = 1:1 ): R_{f}-Wert = 0,69
MS (FD): m/z 544,6 (M⁺)

### 1-D,L-α-Tocopherylglycerol (Toc2)

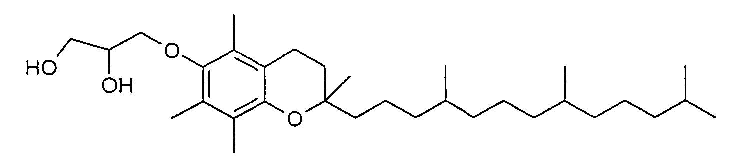

16,9 mmol Toc1 (9,2 g) werden in 100 ml HCl(2 M)/THF (1: 1) gelöst und 2 h bei RT unter Lichtausschluss gerührt. Anschließend wird das Lösungsmittel entfernt, der Rückstand zweimal mit je 650 ml absolutem Ethanol versetzt und das Gemisch wieder zur Trockne eingeengt. Das Rohprodukt wird mit Diethylether/Toluol = 1:1 über Kieselgel säulenchromatographisch aufgerein9gt. Die produkthaltigen Fraktionen werden mit DC identifiziert, vereinigt und zur Trockne eingeengt. Das Produkt wird am Hochvakuum getrocknet und unter Lichtausschluss aufbewahrt. Es resultiert ein gelbes Öl. Ausbeute: 6,6 g (77,5 % der Theorie)
DC (Diethylether/Toluol = 1:1): R_{f}-Wert = 0,22
MS (FD): m/z 504,4 (M⁺)

### [1-(4,4'-Dimethoxytrityl)]-3-D,L-α-tocopherylglycerol (Toc3)

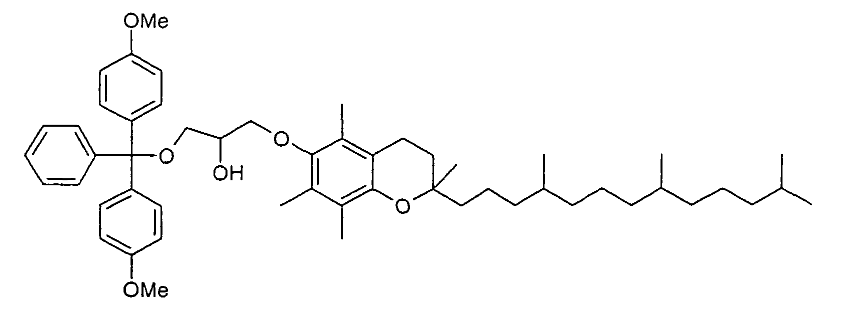

6,6 g Toc2 (13 mmol) werden zum Trocknen zweimal in 16 ml absolutem Pyridin gelöst, das wieder azeotrop abdestilliert wird. Das getrocknete Edukt wird in 60 ml absolutem Pyridin gelöst und mit 5,34 g DMT-Cl (15,8 mmol) versetzt. Nach 12 h Rühren bei RT unter Lichtausschluss wird die Reaktion durch die Zugabe von 50 ml Methanol beendet und das Reaktionsgemisch zur Trockne eingeengt. Der Rückstand wird in 500 ml Dichlormethan aufgenommen und zweimal mit je 150 ml wässriger, gesättigter NaCl-Lösung und anschließend einmal mit 150 ml Wasser gewaschen. Nachdem über Na₂SO₄ getrocknet wurde, wird das Lösungsmittel entfernt und der Rückstand säurenchromatographisch über Kieselgel gereinigt (n-Hexan/Diethylether/Triethylamin = 40:60:1). Die produkthaltigen Fraktionen werden mittels DC identifiziert, vereinigt und zur Trockne eingeengt. Das Produkt wird am Hochvakuum getrocknet. Es resultiert ein gelbliches Öl, das unter Lichtausschluss aufbewahrt wird.
Ausbeute: 8,5 g (81 % der Theorie)
DC (n-Hexan/Diethylether/TEA = 40:60: 1): R_{f}-Wert = 0,43
MS (FD): m/z 807,2

### [1-(4,4'-Dimethoxytrityl)]-2-succinyl-3-D,L-α-tocopherylglycerol (Toc4)

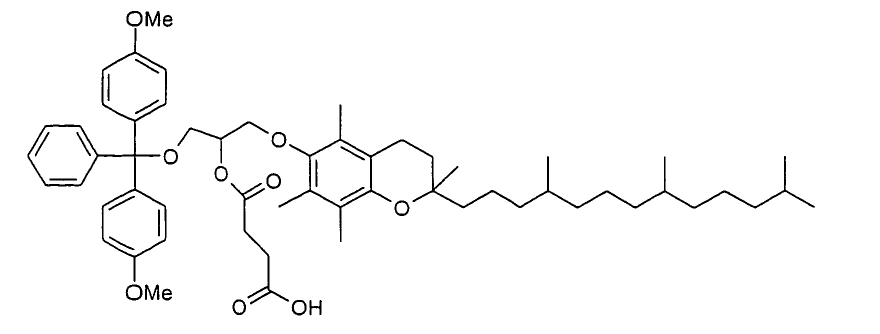

1,45 g Toc3 (1,8 mmol) werden zur Trocknung zweimal in je 6 ml absolutem Pyridin gelöst, das azeotrop wieder abdestilliert wird. Nachdem das Edukt in 8 ml absolutem Pyridin gelöst wurde, gibt man im Argongegenstrom 140 mg DMAP (1,08 mmol) und 194,4 mg Bernsteinsäureanhydrid (1,8 mmol) zu. Anschließend wird 18 h unter Lichtausschluss bei RT gerührt. Zur Aufarbeitung wird die Reaktionslösung mit 45 ml DCM versetzt und viermal mit je 60 ml Wasser gewaschen. Nach Trocknung über Natriumsulfat wird das Lösungsmittel entfernt und das Rohprodukt säurenchromatographisch über Kieselgel mit Ethylacetat/Methanol/NH3 (25 % in H2O) = 5:1:1 gereinigt. Die produkthaltigen Fraktionen werden mittels DC identifiziert, vereinigt und zur Trockne eingeengt. Nach Trocknung im Hochvakuum resultiert ein bräunliches, zähes Öl, das gekühlt und unter Lichtausschluss aufbewahrt wird.
Ausbeute: 1,35 g (82,8 % der Theorie)
DC (EtOAc/NH₃/MeOH = 5:1:1): R_{f}-Wert = 0,30
MS (FD): m/z 906,2 (M⁺), 604,2 (M⁺-DMT)

Beladung von PS-PEG Trägermaterial mit Toc4

Zur Durchführung einer *large scale* Synthese ist eine möglichst hohe Beladung (100 µmol) mit der Ankerverbindung wünschenswert, weshalb mit größeren Mengen Succinat gearbeitet wird als üblich.

Man lässt 1,7 g Tentagel® (0,25 mmol/g) in 15 ml DCM quellen. 310 mg in 10 ml DCM gelöstes Toc4 (341 µmol) werden zum Harz gegeben. 106 mg TBTU werden in ca. 5 ml DMF gelöst und ebenso wie 37 µl NMM zum Reaktionsgemisch gegeben. Die Mischung wird lichtgeschützt 12 h geschüttelt. Anschließend wird das Harz abfiltriert und gründlich mit DMF, DCM und Methanol gewaschen und im Vakuum getrocknet. Um nicht umgesetzte Aminofunktionen zu blockieren, wird ein Capping-Schritt mit je 10 ml der beiden kommerziell erhältlichen Capping-Lösungen (Cap A: Acetanhydrid in THF/Pyridin; Cap B: N-Methylimidazol in THF) durchgeführt (15 min Schütteln). Es wird erneut gewaschen.

Die Beladung wird wie beschrieben über DMT-Abspaltung bestimmt. Es ergibt sich eine Beladung von 138 µmol/g.

### 5'-Tocopheryl-ODN

### D,L-α-Tocopheryl-β-cyanoethyl-N,N-diisopropylphosphoramidit

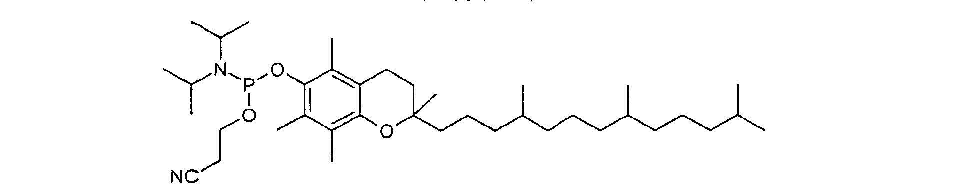

5 g D, L-α-Tocopherol (11,6 mmol) werden zweimal mit je 25 ml Pyridin versetzt und durch azeotropes Schleppen getrocknet. Das Edukt wird in 40 ml DCM_{abs} gelöst. Im Argongegenstrom werden 7,9 ml DIPEA_{abs} (46,4 mmol) und 2,5 ml 2-Cyanoethyl-N,N-diisopropylphosphinchlorid (11 mmol) langsam zugetropft. Nachdem das Reaktionsgemisch 1 h bei RT unter Lichtausschluss gerührt hat, wird es mit 100 ml Ethylacetat/TEA (20:1) verdünnt und zweimal mit 25 ml 10 % NaHCO₃ sowie zweimal mit gesättigter NaCl-Lösung gewaschen. Anschließend wird die organische Phase über Na₂SO₄ getrocknet und das Lösungsmittel am Vakuum entfernt. Das Rohprodukt wird über Kieselgel säulenchromatographisch mit Ethylacetat gereinigt.
Ausbeute: 5,62 g (81 % der Theorie)
DC (EtOAc): R_{f}-Wert = 0,75
MS (FD): m/z 630,1 (M⁺)
³¹p-NMR: δ 152,24

### [1-(4,4'-Dimethoxytrityl)]-(3-D,L-α-tocopheryl)-glycerol-2-phosphoramidit

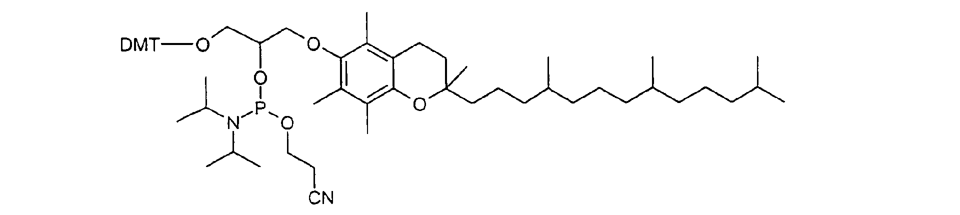

1 g Toc3 (1,24 mmol) wird zur Trocknung zweimal in je 10 ml absolutem Pyridin gelöst, das azeotrop wieder abdestilliert wird. Anschließend löst man das Edukt in 20 ml DCM_{abs} und tropft im Argongegenstrom 0,84 ml DIPEA_{abs} (4,96 mmol) zu. Danach werden 0,27 ml 2-Cyanoethyl-N,N-diisopropylphosphinchlorid (1,19 mmol) im Argongegenstrom langsam zugetropft. Man lässt das Reaktionsgemisch 1 h bei RT rühren, bevor die Lösung mit 50 ml Ethylacetat/TEA (20:1) verdünnt wird. Die organische Phase wird zweimal mit 15 ml einer 10 %igen NaHCO₃-Lösung und zweimal mit einer gesättigten NaCl-Lösung gewaschen und anschließend über Na₂SO₄ getrocknet. Das Lösungsmittel wird entfernt und das Rohprodukt säulenchromatographisch über Kieselgel mit Ethylacetat/TEA (99:1) gereinigt. Die produkthaltigen Fraktionen werden mittels DC identifiziert, vereinigt und zur Trockne eingeengt. Nach Trocknung im Hochvakuum resultiert ein gelbbräunliches, sehr zähes Öl, das gekühlt und unter Lichtausschluss aufbewahrt wird.
Ausbeute: 0,76 g (63,4 % der Theorie)
DC (EtOAc, 1 % TEA): R_{f}-Wert = 0,68
MS (FD): m/z 1006,4 (M⁺), 953,6 (M⁺-Cyanoethyl) 651,4 (M⁺-DMT, -Cyanoethyl), 603,1 (M⁺-DMT, -diisopropylamin), 303,1 (DMT⁺)
³¹ P-NMR: 8 150,5

Das resultierende Tocopherol-modifizierte Phosphoramidit wird dann im Rahmen der Festphasensynthese an das bereits synthetisierte, aber noch an das Trägermaterial gebundene Oligonukleotid angekoppelt.

Auf diese Art und Weise wurden folgende Oligonukleotide synthetisiert:

MON1: 5'-Tocopheryl-CCT GTT GTT TTC TCT-3'-Tocopheryl (SEQ ID NO:1);

MON2: 5'-Tocopheryl-CCT CGC TCC GCC CGT-3'-Tocopheryl (SEQ ID NO:2);

MON3: 5'-Tocopheryl-TGC TCC CCC CTG GCT-3'-Tocopheryl (SEQ ID NO:3).

Weiterhin konnte gezeigt werden, dass trotz der hoch effektiven Proliferationshemmung humaner Pankreastumorzellen durch MON1 nichtmaligne Zellen wie humane Fibroblasten in ihrem Wachstum im Wesentlichen nicht beeinflusst wurden.

### Zellkulturen und Zellaufnahme

Als humane Pankreastumorzellen wurden vor allem Panc-Tu-I-, Panc-Tu-II-, A818-4 und HPAF-Zelllinien verwendet. Die Aufnahme der modifizierten ODN durch die Zellen wurde mit konfokaler Lasermikroskopie bestimmt.

### Messung der Proliferation in vitro

Zellsuspensionen (7 x 10⁴ Zellen ml⁻¹) wurden in jede Kavität einer Mikrotiterplatte mit 96 Kavitäten (Nunc, Wiesbaden) gegeben. Nach 24 Stunden wurde das Medium gewechselt und die Zellkulturen (mit der jeweiligen ODN-Behandlung MON1, MON2 oder MON3 und die Kontrollen ohne Behandlung) wurden in 100 µl Ansätzen mit 10 µl Medium, das 7.4 kBq Methyl-³H-Thymidin (Amersham, Braunschweig) enthielt, während der letzten 3 Stunden der angegebenen Inkubationszeiten markiert. Anschließend wurden die Zellen geerntet und die Radioaktivität mit einem Flüssig-Szintillationszähler gemessen.

### In vivo-Untersuchungen an SCID-Mäusen

Es wurden weibliche SCID Mäuse verwendet, die nicht älter als acht Wochen waren. Den Tieren wurden nach Laparotomie in Narkose 1 x 10⁶ PancTu-1 Zellen in das Pankreas injiziert. Die Mäuse wurden in fünf Behandlungsgruppen sowie eine Kontrollgruppe zu je sechs Tieren eingeteilt. Den Mäusen der Kontrollgruppe wurde ab dem 7. Tag täglich 500 µl PBS intraperitoneal injiziert, den Mäusen der Behandlungsgruppen MON1 in verschiedenen Dosierungen und Intervallen: Behandlungsgruppe 1 wurde in Intervall A (abwechselnd drei Tage Behandlung und drei Tage Pause) mit 6 mg/kg Körpergewicht intraperitoneal behandelt, Behandlungsgruppe 2 mit gleicher Dosierung in Intervall B (6 Tage Behandlung, 12 Tage Pause im Wechsel). Behandlungsgruppe 3 erhielt die gleiche kumulative Dosis wie die Behandlungsgruppen 1 und 2 (72 mg/kg Körpergewicht), allerdings durch tägliche Applikation von 3 mg/kg Körpergewicht intraperitoneal. Behandlungsgruppe 4 wurde ebenfalls mit 3 mg/kg Körpergewicht behandelt, jedoch in Intervall A mit einer kumulativen Dosis von 36 mg/kg Körpergewicht. Behandlungsgruppe 5 wurde mit einer täglichen Dosis von 1 mg/kg Körpergewicht und einer kumulativen Dosis von 24 mg/kg Körpergewicht behandelt. Alle Gruppen wurden über einen Zeitraum von 24 Tagen behandelt.

Die Mäuse wurden nach 24 Tagen getötet. Die Pankreastumoren wurden gewogen und vermessen, um das Volumen nach der Formel Π/6x Höhe x Länge x Breite zu berechnen. Auch das Gewicht des gesamten Tumorgewebes wurde bestimmt. Leber, Lungen, Omentum und Milz wurden entnommen, um typische Metastasierungswege zu erfassen. Aus der Differenz zwischen den Gewichten der entnommenen Pankreastumoren und den Gewichten des gesamten Tumorgewebes ist ersichtlich, dass das verwendete Tiermodell nicht nur auf einem Primärtumor basierte, sondern durchaus auch Metastasen im Bauchraum auftraten.

### Hemmung der Proliferation in vitro

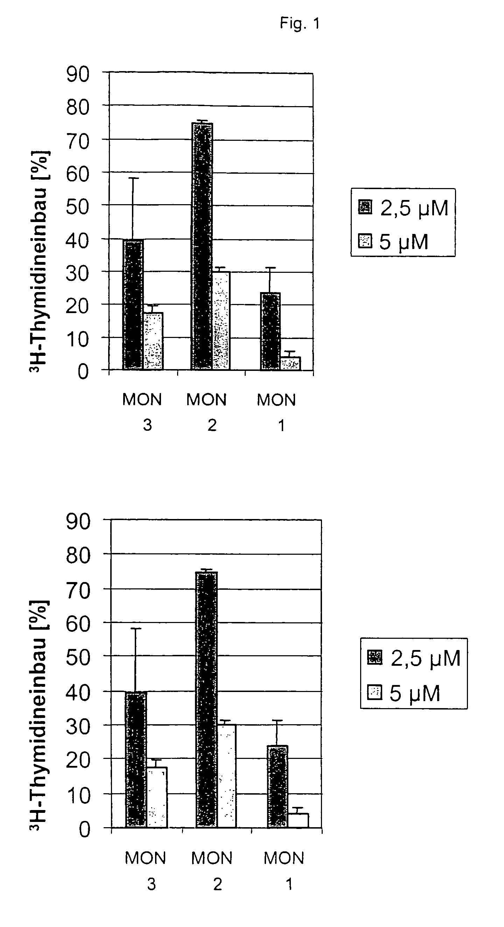

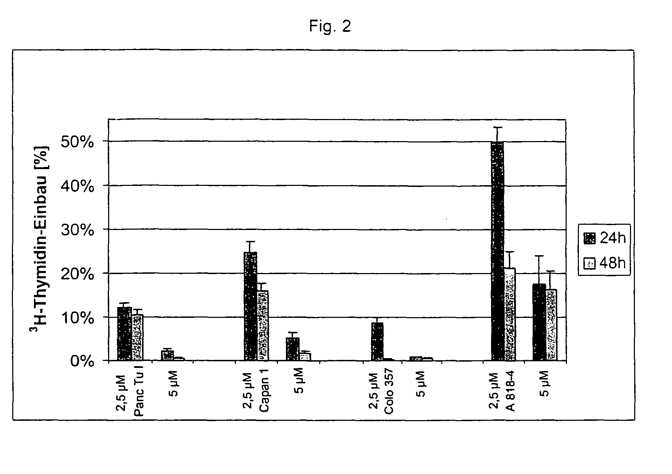

Die proliferationshemmende Wirkung des Oligonukleotids MON1 war der proliferationshemmenden Wirkung der Oligonukleotide MON2 und MON3 deutlich überlegen. Dies zeigte sich sowohl im ³H-Proliferations-Assay (Figur 1). Ferner war zu beobachten, dass die Wirkung zeit- und dosisabhängig war. Mit MON 1 war auch bei der geringeren Dosierung von 2,5 µM bereits nach 24 Stunden eine deutliche Proliferationshemmung auf ca. 25 % relativ zur unbehandelten Kontrolle zu beobachten. Betrug die Dosis 5 µM ging das Wachstum der humanen Pankreastumorzellen Panc-Tu-I im Vergleich zur unbehandelten Kontrolle auf ca. 4 % zurück. Die Oligonukleotide MON2 und MON3 zeigten eine deutlich weniger stark ausgeprägte Wachstumshemmung. Die stark proliferationshemmende Wirkung des Oligonukleotids MON1 konnte nicht nur an Zellen der Panc-Tu1-Zelllinie, sondern auch an weiteren Pankreaskarzinom-Zelllinien (Capan1; Colo357; A818-4) unter den vorstehend beschriebenen Bedingungen beobachtet werden (Figur 2).

### Hemmung der Proliferation in vivo

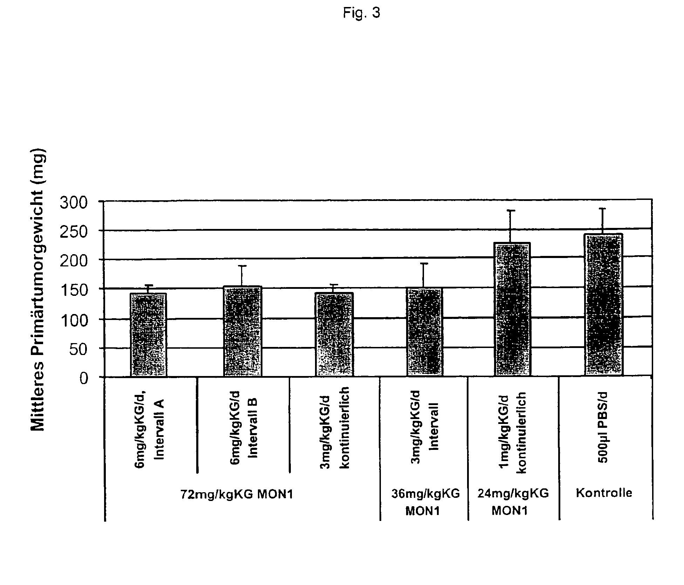

Die Primärtumoren der behandelten Mäuse wogen nach 24 Tagen Therapie zwischen 59 % und 64 % des durchschnittlichen Primärtumorgewichts der unbehandelten Mäuse. Lediglich bei Behandlungsgruppe 5 (kontinuierlich 1 mg/kg Körpergewicht MON1) konnte keine Veränderung im Vergleich zur Kontrollgruppe festgestellt werden. Die besten Resultate zeigten die Behandlungsgruppen 1 (6 mg/kg Körpergewicht pro Tag, 3 Tage Therapie, 3 Tage Pause) und 3 (kontinuierlich 3 mg/kg Körpergewicht pro Tag) mit einem jeweils auf 59 % reduzierten Tumorwachstum (Figur 3).

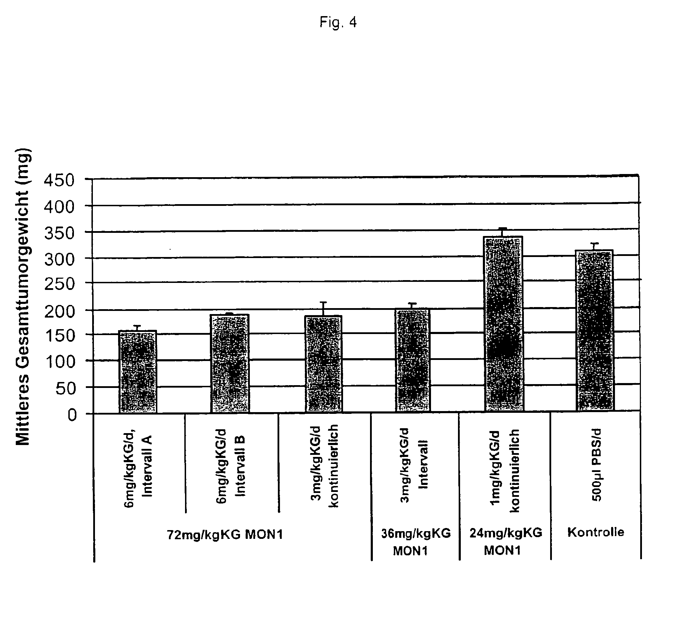

Ein ähnliches Bild zeigte sich bei der Untersuchung des Gesamtrumorgewichts (Figur 4). Hier bringt die hohe Dosierung (6 mg/kg Körpergewicht pro Tag bei gleichmäßigen Therapieintervallen (3 Tage Therapie, 3 Tage Pause) die besten Resultate. In Behandlungsgruppe 1 war das Wachstum des Gesamttumorgewebes um 50 % zurückgegangen. Die restlichen Behandlungsgruppen zeigten mit einem Wachstumsrückgang auf 59-63 % ähnliche Resultate wie bei der Auswertung des Primärtumorgewichts. Ein geringfügig schlechteres Ergebnis (63 %) erhielt man bei der Intervallbehandlung mit 3 mg/kg Körpergewicht pro Tag. Behandlungsgruppe 5 (kontinuierlich täglich 1 mg/kg Körpergewicht) zeigte wiederum praktisch keinen Unterschied zur Kontrollgruppe.

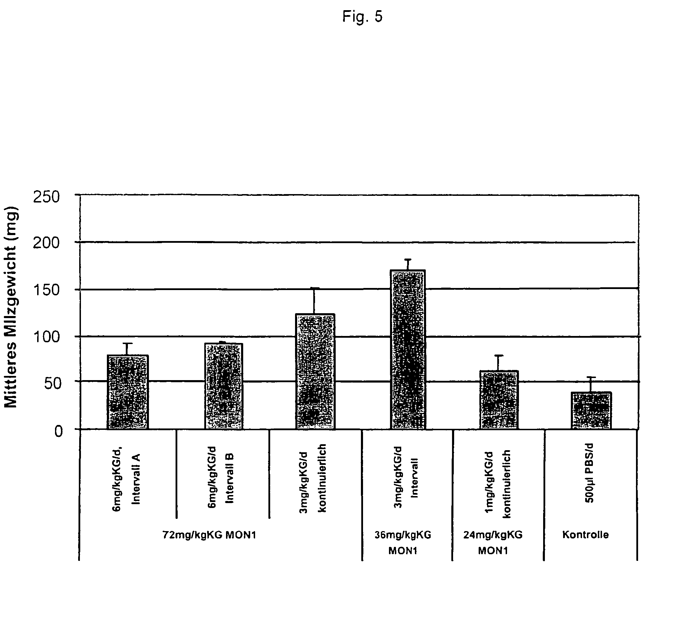

Die pathologische Untersuchung der Mausorgane ergab abgesehen von den üblichen postmortalen Erscheinungen keine Besonderheiten, d.h. sie waren durch die MON-Behandlung nicht geschädigt. Die Milz der behandelten Tiere war in allen Versuchsgruppen deutlich vergrößert (Figur 5). Die geringste Veränderung war in Behandlungsgruppe 5 (kontinuierlich 1 mg/kg Körpergewicht pro Tag) mit einer 50 %igen Gewichtszunahme zu finden. Allerdings war in dieser Gruppe das Tumorwachstum auch kaum gehemmt. Die Milz der Tiere der Behandlungsgruppe 4 war die schwerste, ihr Gewicht hatte sich in Bezug auf die unbehandelte Kontrolle mehr als vervierfacht. Sonstige Nebenwirkungen waren bis auf einen reversiblen Rückgang der Leukozyten und einer reversiblen leichten Thrombozytopenie, die bei Behandlung mit polyanionischen Molekülen oft beschrieben wird, nicht zu beobachten.

### Annex zu den Anmeldungsunterlagen - nachgereichtes Sequenzprotokoll

## Patentansprüche

1. Oligonukleotid mit der Sequenz SEQ ID NO: 1.

2. Oligonukleotid, **dadurch gekennzeichnet, dass** das Oligonukleotid eine Anordnung von wenigstens 6 konsekutiven Nukleobasen aus der Sequenz SEQ ID NO: 1 aufweist.

3. Oligonukleotid, **dadurch gekennzeichnet, dass** wenigstens ein Teil der Nukleobasen entsprechend der Sequenz SEQ ID NO: 1 angeordnet ist.

4. Oligonukleotid nach einem der vorhergehenden Ansprüche, **dadurch gekennzeichnet, dass** mindestens ein Thymidinrest oder ein Mimetikum davon in konsekutiver Abfolge zu einem Guanin oder einem Mimetikum davon angeordnet ist.

5. Oligonukleotid nach einem der vorhergehenden Ansprüche, **dadurch gekennzeichnet, dass** weniger als 15 % der Nukleobasen Adenin oder Mimetika davon sind.

6. Oligonukleotid nach einem der vorhergehenden Ansprüche, **dadurch gekennzeichnet, dass** das Oligonukleotid 8 bis 30 Nukleobasen aufweist.

7. Oligonukleotid nach einem der vorhergehenden Ansprüche, **dadurch gekennzeichnet, dass** das Oligonukleotid am 3'- und/oder 5'-Terminus modifiziert ist.

8. Oligonukleotid nach Anspruch 7, **dadurch gekennzeichnet, dass** terminal wenigstens ein Polyalkylenglykol, ein lipophiler Rest oder ein Peptid gebunden ist.

9. Oligonukleotid nach Anspruch 8, **dadurch gekennzeichnet, dass** der lipophile Rest ein Fettsäurerest, ein Steroidrest, oder ein Tocopherylrest oder ein Derivat dieser Reste ist.

10. Oligonukleotid nach einem der Ansprüche 7 bis 9, **dadurch gekennzeichnet, dass** der eine Terminus einen Tocopherylrest und der andere Terminus einen Tocopherylrest oder einen Polyethylenglykolrest aufweist.

11. Oligonukleotid nach Anspruch 10, **dadurch gekennzeichnet, dass** der Tocopherylrest am 5'-Terminus und der Polyethylenglykolrest am 3'-Terminus gebunden ist.

12. Oligonukleotid nach einem der vorhergehenden Ansprüche, **dadurch gekennzeichnet, dass** das Oligonukleotid wenigstens eine Phosphothioat-Verknüpfung aufweist.

13. Verwendung eines Oligonukleotids nach einem der Ansprüche 1 bis 12 zur Herstellung eines pharmazeutischen Mittels zur Behandlung von Tumoren, insbesondere Pankreastumoren.


SEQUENZPROTOKOLL

<110> KALTHOFF, Holger
<120> Oligonukleotide, diese enthaltende Mittel und deren
Verwendung
<130> P 5395

<140> EP 04 024 018.6
<141> 2004-10-08

<160> 3

<170> PatentIn version 3.1

<210> 1
<211> 15
<212> DNA
<213> Künstliche Sequenz

<400> 1
cctgttgttt tctct 15

<210> 2
<211> 15
<212> DNA
<213> Künstliche Sequenz

<400> 2
cctcgctccg cccgt 15

<210> 3
<211> 15
<212> DNA
<213> Künstliche Sequenz

<400> 3
tgctcccccc tggct 15